# EUROPEAN PATENT APPLICATION

(11) **EP 2 339 352 A1**
(43) Date of publication of application: **29.06.2011**
(21) Application number: 09180820.4
(22) Date of filing: 28.12.2009
(51) Int. Cl.: G01N 33/92

(54) **Use of endogenous metabolites for early diagnosing sepsis**

(71) Applicant: BIOCRATES Life Sciences AG, 6020 Innsbruck (AT)
(72) Inventor: Deigner, Hans-Peter, 68623 Lampertheim (DE); Enot, David, 14480 Creully (FR); Kohl, Matthias, 95326 Kulmbach (DE); Igwe, Emeka, 6090 Innsbruck (AT); Koal, Therese, 6020 Innsbruck (AT); Bauer, Michael, 07747 Jena (DE); Dallmann, Guido, 6020 Innsbruck (AT)
(74) Representative: Winter, Brandl, Fürniss, Hübner, Röss, Kaiser, Polte - Partnerschaft

(57) **Abstract**

The present invention relates to a use of one or a plurality of endogenous target metabolites for predicting a likelihood of an onset of a sepsis from a biological sample of a mammalian subject in vitro, wherein said endogenous target metabolites have a molecular mass less than 1500 Da and are selected from the group of oxysterols.

## Description

The present invention relates to a use of on or a plurality of endogenous target metabolites for predicting a likelihood of an onset of a sepsis from a biological sample of a mammalian subject *in vitro* in accordance with claim 1.

The invention generally relates to biomarkers for sepsis as tools in clinical diagnosis for early detection of sepsis, therapy monitoring and methods based on the same biomarkers.

### BACKGROUND of the Invention

The early detection of a disease condition allows for a more effective therapeutic treatment with a correspondingly more favourable clinical outcome. In many cases, however, early detection of disease symptoms is problematic since the disease may have progressed before diagnosis is possible. Systemic inflammatory conditions represent one such class of diseases. These conditions, particularly sepsis, typically result from an interaction between a pathogenic microorganism and the host's defense system that triggers an excessive and dysregulated inflammatory response in the host. The complexity of the host's response during the systemic inflammatory response has complicated efforts towards understanding disease pathogenesis. (Reviewed in Healy, Annul. Pharmacother. 36: 648-54 (2002).) An incomplete understanding of the disease pathogenesis, in turn, contributes to the difficulty in finding diagnostic biomarkers. Early and reliable diagnosis is imperative, however, because of the remarkably rapid progression of sepsis into a life-threatening condition.

Sepsis is a common cause of mortality and morbidity worldwide. In a recent evaluation of the epidemiology of severe sepsis in children in the United States, the estimated annual incidence of severe sepsis in newborns was 0.3 per 100 live births (Watson et al., 2003) with most mortality occurs within the first 48 hours of infection (Weinschenk et al., 2000; Stoll et al., 2002).

Clinically, sepsis is a term used to describe symptomatic bacteremia, with or without organ dysfunction. Sustained bacteremia, in contrast to transient bacteremia, may result in a sustained febrile response that may be associated with organ dysfunction. Septicemia refers to the active multiplication of bacteria in the bloodstream, leading to an overwhelming infection. The pathophysiology of sepsis is complex and the roles of inflammation, coagulation, and suppressed fibrinolysis are emerging as important mechanisms in the pathophysiology of sepsis. These mediators of inflammation are often responsible for the clinically observable effects of the bacteremia in the host. Furthermore, impaired pulmonary, hepatic, or renal function may result from excessive release of inflammatory mediators during a septic process.

Sepsis by definition comprises Systemic inflammatory response syndrome and infection with pathogens.

Systemic inflammatory response syndrome (SIRS) is considered to be present when two or more of the following clinical findings are present:
1. Body temperature >38°C or <36°C;
2. Heart rate >90 min-1;
3. Hyperventilation evidenced by a respiratory rate of >20 min-1 or a PaCO2 of <32 mm Hg; and
4. White blood cell count of >12,000 cells µL-1 or <4,000 µL-1

Sepsis includes a systemic inflammatory response syndrome (SIRS) together with an infection.

Sepsis (commonly called a "blood stream infection) denotes the presence of bacteria (bacteremia) or other infectious organisms or their toxins in the blood (septicemia) or in other tissue of the body and the immune response of the host. Sepsis is currently thought to result from the interaction between the host response and the presence of micro-organisms and/or their toxins within the body. The observed host responses include immune, coagulation, pro- and anti-inflammatory responses. Sepsis thus comprises a systemic response to infection, defined as hypothermia or hyperthermia, tachycardia, tachypnea, a clinically evident focus of infection or positive blood cultures, one or more end organs with either dysfunction or inadequate perfusion, cerebral dysfunction, hypoxaemia, increased plasma lactate or unexplained metabolic acidosis, and oliguria.

While usually related to infection, it can also be associated with noninfectious insults such as trauma, burns, and pancreatitis. It is one of the most common causes of adult respiratory distress syndrome.

A precise definition of the term sepsis has been introduced by the ACCP/SCCM Consensus Conference Committee (1992): Definition for sepsis and organ failure and guidelines for the use of innovative therapies in sepsis. Crit Care Med. 20(6):864-874.The 2001 International Sepsis Definitions Conference attempted to improve the above definition with the aim of increasing the accuracy of the diagnosis of sepsis Levy M, Fink M, Mitchell P, Marshall JC, Abraham E, et al. For the International Sepsis Definitions Conference. 2001 SCCM/ESICM/ACCP/ATS/SIS. The statement suggested that although the SIRS concept was valid, in the future if supported by further epidemiologic data, it may be possible to use purely biochemical and/or immunologic, rather than clinical criteria to identify the inflammatory response. It also defined infection as a pathologic process induced by a micro-organism, and that sepsis should be defined as a patient with documented or suspected 'infection' exhibiting some of the following variables:
1. General variables
   o Fever (core temperature >38.3°C)
   o Hypothermia (core temperature <36°C)
   o Heart rate >90 min-1 or >2 SD above the normal value for age
   o Tachypnea
   o Altered mental status
   o Significant oedema or positive fluid balance (>20 mL/kg over 24 hrs)
   o Hyperglycemia (plasma glucose >7.7 mmol/L) in the absence of diabetes
2. Inflammatory variables
   o Leukocytosis - WBC count >12,000 µL-1
   o Leukopaenia - WBC count <4000 µL-1
   o Normal WBC count with >10% immature forms
   o Plasma C-reactive protein >2 SD above the normal value
   o Plasma procalcitonin >2 SD above the normal value
3. Hemodynamic variables
   o Arterial hypotension (SBP <90 mmHg, MAP <70 mmHg, or an SBP decrease >40 mmHg in adults)
   o SvO2a >70%
   o Cardiac index > 3.5 Lmin-1 M-2
4. Organ dysfunction variables
   o Arterial hypoxemia (PaO2/FIO2 <300)
   o Acute oliguria (urine output <0.5 mLkg-1 hr-1 for at least 2hrs)
   o Creatinine increase >0.5 mg/dL
   o Coagulation abnormalities (INR >1.5 or aPTT >60 secs)
   o Ileus (absent bowel sounds)
   o Thrombocytopenia (platelet count <1 00µL)
   o Hyperbilirubinemia (plasma total bilirubin>4 mg/dL or 70 mmol/L)
5. Tissue perfusion variables
   o Hyperlactatemia (>1 mmol/L)
   o Decreased capillary refill or mottling
(WBC, white blood cell; SBP, systolic blood pressure; MAP, mean arterial blood pressure; SvO2, mixed venous oxygen saturation; INR, international normalized ratio; aPTT, activated partial thromboplastin time; tachycardia (may be absent in hypothermic patients)), and at least one of the following indications of altered organ function: altered mental status, hypoxemia, increased serum lactate level, or bounding pulses.

The definition of severe sepsis remained unchanged and refers to sepsis complicated by organ dysfunction. Organ dysfunction is defined using Multiple Organ Dysfunction score Marshall JC, Cook DJ, Christou NV, et al. Multiple organ dysfunction score: A reliable descriptor of a complex clinical outcome. Crit Care Med 1995; 23: 1638―1652 or the definitions used for the Sequential Organ Failure Assessment (SOFA) score Ferreira FL, Bota DP, Bross A, et al. Serial evaluation of the SOFA score to predict outcome in critically ill patients. JAMA 2002; 286: 1754―1758. Septic shock in adults refers to a state of acute circulatory failure characterized by persistent arterial hypotension unexplained by other causes. Hypotension is defined by a systolic arterial pressure below 90 mm Hg, a MAP <70 mmHg, or a reduction in systolic blood pressure of >40 mm Hg from baseline, despite adequate volume resuscitation, in the absence of other causes for hypotension.

The mortality rate associated with sepsis, severe sepsis and septic shock are high and reported as 25 to 30% and 40 to 70% respectively. Bernard GR, Vincent JL, Laterre PF, et al. Efficacy and safety of recombinant human activated protein C for severe sepsis. N Engl J Med 2001; 344: 699―709. Annane D, Aegerter P, Jars-Guincestre MC, Guidet B. Current epidemiology of septic shock: the CUB-Rea Network. Am J Respir Crit Care Med 2003; 168: 165-72.

Despite some advances in the management of severe sepsis and septic shock, problems remain regarding the usefulness of the currently used definitions and the often encountered delays in diagnosis. The reliable diagnosis of sepsis still remains a challenge. The diagnostics of sepsis and SIRS so far relies on clinical criteria and biochemical parameters limited to protein markers such as procalcitonin (PCT), C reactive protein (CRP) and interleukins (Beger HG, Rau BM, Severe acute pancreatitis: Clinical course and management World J Gastroenterol. 2007, 13, 5043-51).

Besides antibiotics therapy, the treatment of sepsis is still limited to preventive measures and symptomatic supportive strategies.

Current diagnostics in clinical routine is limited to a) clinical information b) use of basic biochemical as outlined below in the definitions.

Or the use of unspecific biomarkers like C-reactive protein (CRP) or procalcitonin (PCT) with low sensitivities and specificities (Carrol et al., 2002; Toikka et al., 2000; Gendrel et al., 1997, Tang et al., 2007). Moreover, the levels of these biomarker are also raised in many non sepsis-associated diseases like in small-cell lung cancer, medullary thyroid cancer and inhalation injury (Becker et al., 1993).

The identification, let alone the quantification of pathogens or of nucleic acids from these pathogens in an ill subject is far from being reliable, validated or sufficient for diagnosis, a large body of scientific evidence supports diagnostics based on the molecular response and immune response of the host, actually reflecting the individual clinical state of the subject, regardless of the nature or quantities of the underlying pathogens, respectively fragments of these organisms.

In classical patient screening and diagnosis, the medical practitioner uses a number of diagnostic tools for diagnosing a patient suffering from a certain disease. Among these tools, measurement of a series of single routine parameters, e.g. in a blood sample, is a common diagnostic laboratory approach. These single parameters comprise for example enzyme activities and enzyme concentration and/or detection

As far as such diseases are concerned which easily and unambiguously can be correlated with one single parameter or a few number of parameters achieved by clinical chemistry, these parameters have proved to be indispensable tools in modern laboratory medicine and diagnosis. However, in pathophysiological conditions, such as cancer or demyelinating diseases such as multiple sclerosis which share a lack of an unambiguously assignable single parameter or marker, differential diagnosis from blood or tissue samples is currently difficult to impossible.

Although nucleic acid-based diagnosis of sepsis from blood cells has been explored recently (Yadav et al., 2008; Andrade et al., 2008; Tang et al., 2008, 2009), these approaches, however, suffer from several serious limitations:
The required sample size of usually several ml of blood is a problem for continuous monitoring of a critically ill subject; alternatives applying amplification of transcripts are lengthy and prone to error. The whole procedure affords numerous steps and due to laborious sample preparation and nucleic acid isolation, transcription and array or PCR analysis still takes at least several hours and a large technological effort.

Currently used diagnostic methods thus require time and appropriate equipment with high costs and frequently unsatisfying sensitivities. However these used diagnostic means have major limitations either to reduced area under the curve (AUC) and/or delay of diagnosis or increased costs due to equipment required. Accordingly these procedures do not allow a timely assessment of an acute and rapidly evolving disease and overall the situation is far from satisfying and from providing a rapid and reliable diagnosis of sepsis.

Furthermore, a number of metabolomic approaches for characterizing sepsis with poor prognostic and clinical value have been made with different groups of metabolites, such as fatty acids (Ping-bo Xu et al. (2008), Journal of Infection 56, 474e481); lysophosphatidylcholines (US 20070111316); unsaturated free fatty acids (US 5,780,237); carnitine and acylcarnitines (G. Famularo et al. (1995), Journal of Endotoxin Research, Vol. 2, No. 2, 141-147).

However, none of them provided a convincing and reliable method of diagnosis for the early phases of sepsis, and determination of the likelihood of an onset of severe sepsis and septic shock.

Therefore, there is still an urgent need for an early, rapid and reliable diagnosis of sepsis, differentiation of sepsis and SIRS or any other state of health providing the unspecific clinical symptoms listed in the definition of sepsis as well as sepsis severity, ideally requiring only minute amounts of blood; there is an urgent need for timely treatment and early diagnosis of sepsis as well as, an urgent need for therapy monitoring. Further, there is an urgent need for early sepsis biomarkers enabling early and reliable diagnosis, risk stratification of sepsis per se, sepsis duration and severity.

Thus, the problem to be solved with the present invention was to provide an alternative and more reliable diagnostic metabolomic approach for predicting an onset of sepsis, and sepsis-related conditions.

The above problem is solved by a use of endogenous target metabolites from the oxysterol type in accordance with claim 1.

In particular, the present invention provides a solution to these problems based on the application of a new technology in this context and on an unknown list of endogenous metabolites of the oxysterol type as diagnostic marker. Since metabolite concentration differences in biological fluids and tissues provide links to the various phenotypical responses, metabolites are suitable biomarker candidates.

In more detail, the present invention relates to the use of one or a plurality of endogenous target metabolites for predicting a likelihood of an onset of a sepsis from a biological sample of a mammalian subject in vitro, wherein

said endogenous target metabolites have a molecular mass less than 1500 Da and are selected from the group consisting of:
oxysterols, namely cholesterol, 20α-hydroxycholesterol, 22(R)-hydroxycholesterol, 22(S)-hydroxycholesterol, 24(S)-hydroxycholesterol, 25-hydroxycholesterol, 27-hydroxycholesterol, 24(R),25-epoxycholesterol, 24(S),25-epoxycholesterol, 3ß,5α,6ß-trihydroxycholestan, 7α-hydroxycholesterol, 7-Ketocholesterol, 5α,6α- epoxycholesterol 5ß,6ß-epoxycholesterol, 4ß-hydroxycholesterol, desmosterol, 7-dehydrocholesterol, cholestenone, lanosterol; 24,25-dihydrolanosterol.

The present invention allows for accurate, rapid, and sensitive prediction and diagnosis of sepsis through a measurement of one or plurality of endogenous metabolic biomarker (metabolites) taken from a biological sample at a single point in time. This is accomplished by obtaining a biomarker panel at a single point in time from an individual, particularly an individual at risk of developing sepsis, having sepsis, or suspected of having sepsis, and comparing the biomarker profile from the individual to reference biomarker values or scores. The reference biomarker values may be obtained from a population of individuals (a "reference population") who are, for example, afflicted with sepsis or who are suffering from either the onset of sepsis or a particular stage in the progression of sepsis. If the biomarker panel values or score from the individual contains appropriately characteristic features of the biomarker values or scores from the reference population, then the individual is diagnosed as having a more likely chance of getting sepsis, as being afflicted with sepsis or as being at the particular stage in the progression of sepsis as the reference population.

Accordingly, the present invention provides, inter alia, methods of predicting the likelihood of an onset of sepsis in an individual. The methods comprise obtaining a biomarker score at a single point in time from the individual and comparing the individual's biomarker profile to a reference biomarker profile. Comparison of the biomarker profiles can predict the onset of sepsis in the individual preferably with an accuracy of at least about 90%. This method may be repeated again at any time prior to the onset of sepsis.

A preferred embodiment of the present invention is characterized in that the subject's quantitative metabolomics profile comprising one or a plurality of said endogenous metabolites, is detected in the biological sample by means of quantitative metabolomics analysis, and the quantitative metabolomics profile of the subject's sample is compared with a quantitative reference metabolomics profile of a plurality of endogenous sepsis target metabolites in order to predict whether the subject is likely or unlikely to develop a sepsis.

For easier and/or more sensitive detection, said endogenous target metabolites may be detected by means of chemically modified derivatives thereof, such as acylated, silylated derivatives, or as hydrazones, in particular, Girard P hydrazones, mono- or di-dimethylglycine esters or picolinyl esters of said target metabolites.

Preferably, the one or a plurality of the following target metabolites are used for an early diagnosis of sepsis:
22(R)-hydroxycholesterol, 24(S)-hydroxycholesterol, 25-hydroxycholesterol, 27-hydroxycholesterol, 24,25 epoxycholesterol, 7α-hydroxycholesterol, 4ß-hydroxycholesterol, desmosterol, 7-dehydrocholesterol, cholestenone, lanosterol, 24,25-dihydrolanosterol.

For an early diagnosis of a severe sepsis said one or a plurality of endogenous target metabolites are selected from the group consisting of:
22(R)-hydroxycholesterol, 24(S)-hydroxycholesterol, 25-hydroxycholesterol, 27-hydroxycholesterol, 3ß,5α,6ß-trihydroxycholestan, 7α-hydroxycholesterol, 7-ketocholesterol, 5ß,6ß-epoxycholesterol, 5α,6α-epoxycholesterol, 4ß-hydroxycholesterol, desmosterol, 7-dehydrocholesterol, cholestenone, lanosterol, 24,25-dihydrolanosterol.

For the prediction of a likelihood of an onset of a septic shock it is preferred to chose such endogenous target metabolites which are selected from the group consisting of:
24(S)-hydroxycholesterol, 25-hydroxycholesterol, 27-hydroxycholesterol, 7-ketocholesterol, 5ß,6ß-epoxycholesterol, 5α,6α-epoxycholesterol, 4ß-hydroxycholesterol, desmosterol, lanosterol.

In accordance with a preferred embodiment of the present invention, the biological sample is selected from the group consisting of stool; body fluids, in particular blood, liquor, cerebrospinal fluid, urine, ascitic fluid, seminal fluid, saliva, puncture fluid, cell content, tissue samples, in particular liver biopsy material; or a mixture thereof.

Preferably the quantitative metabolomics profile is achieved by a quantitative metabolomics profile analysis method comprising the generation of intensity data for the quantitation of endogenous metabolites by mass spectrometry (MS), in particular, by high- throughput mass spectrometry, preferably by MS-technologies such as Matrix Assisted Laser Desorption/lonisation (MALDI), Electro Spray Ionization (ESI), Atmospheric Pressure Chemical Ionization (APCI), 1H-, 13C- and/or 31 P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).

The present invention further provides a method of determining the progression (i.e., the stage) of sepsis in an individual. This method comprises obtaining a biomarker profile at a single point in time from the individual and comparing the individual's biomarker profile to a reference biomarker score. Comparison of the biomarker scores can determine the progression of sepsis in the individual preferably with an accuracy of at least about 90 %. This method may also be repeated on the individual at any time.

Additionally, the present invention provides a method of diagnosing sepsis in an individual having or suspected of having sepsis. This method comprises obtaining a biomarker score at a single point in time from the individual and comparing the individual's biomarker score to a reference biomarker score. Comparison of the biomarker profiles can diagnose sepsis in the individual with an accuracy of at least about 90 %. This method may also be repeated on the individual at any time.

In another embodiment, the invention provides, inter alia, a method of determining the status of sepsis or diagnosing sepsis in an individual comprising applying a decision rule. The decision rule comprises comparing (i) a biomarker score generated from a biological sample taken from the individual at a single point in time with (ii) a biomarker score generated from a reference population. Application of the decision rule determines the status of sepsis or diagnoses of sepsis in the individual. The method may be repeated on the individual at one or more separate, single points in time.

The present invention further provides, inter alia, a method of determining the status of sepsis or diagnosing sepsis in an individual comprising obtaining a biomarker score from a biological sample taken from the individual and comparing the individual's biomarker score to a reference biomarker score. A single such comparison is capable of classifying the individual as having membership in the reference population. Comparison of the biomarker scores determines the status of sepsis or diagnoses of sepsis in the individual.

In yet another embodiment, the present invention provides, inter alia, a method of determining the status of sepsis or diagnosing sepsis in an individual. The method comprises comparing a measurable characteristic of at least one biomarker between a biomarker panel or biomarker score composed by (processed or unprocessed) values of this panel obtained from a biological sample from the individual and a biomarker score obtained from biological samples from a reference population. Based on this comparison, the individual is classified as belonging to or not belonging to the reference population. The comparison, therefore, determines the likelihood of sepsis or diagnoses of sepsis in the individual. The biomarkers, in particular the endogenous target metabolite used in the present invention, are selected from the group of biomarkers shown in any one of TABLES 1 to 4.

The present invention provides methods for predicting sepsis. Such methods comprise the steps of: analyzing a biological sample from a subject to determine the level(s) of one or more biomarkers for sepsis in the sample, where the one or more biomarkers are selected from Table 1 and comparing the level(s) of the one or more biomarkers, as well as a composed value / score generated by subjecting the concentrations of individual biomarkers in the sample to a classification method such as affording an equation to process single concentration values - to obtain a separation between both (diseased and healthy) groups or comparing the level(s) of the one or more biomarkers in the sample to sepsis positive or sepsis negative reference levels of the one or more biomarkers in order to determine whether the subject is developing sepsis.

The present invention provides a solution to the problem described above, and generally relates to the use of metabolomics data, generated by quantitation of endogenous metabolites by but not limited to mass spectrometry (MS), in particular MS-technologies such as MALDI, ESI, atmospheric pressure pressure chemical ionization (APCI), and other methods, determination of metabolite concentrations by use of MS-technologies or alternative methods coupled to separation (LC-MS, GC-MS, CE-MS), subsequent feature selection and /or the combination of features to classifiers including molecular data of at least one molecules.

The concentrations of the individual markers, analytes, metabolites thus are measured and compared to reference values or data combined and processed to scores, classifiers and compared to reference values thus indicating diseased states etc. with superior sensitivities and specificities compared to known procedures, clinical parameters and biomarkers.

Those skilled in the art will understand that for the quantitation of certain metabolites, also chemically modified metabolites may be used. For example, it is a well established practice to use acylation or silylation as modifying reaction, which convert compounds with active hydrogens, such as OH-, SH and NH- groups e.g. into esters, thioesters and amides, respectively, in case of acylation. Such derivatization often results in a more sensitive (sensitivity enhancement up to 100 fold) and preciser quantification, as one gets a better separation on the column material used prior to the MS-technologies.

However, as e.g. ESI is not necessarily the best ionization method for neutral steroids because of its rather poor ionization efficiency. In such cases, derivatization could markedly enhance the ionization efficiency in the ESI process.

Common derivatives for oxysterols are described in literature:
● Girard P hydrazone (Griffiths W. J., Wang Y., Alvelius G., Liu S., Bodin K., Sjövall J. (2006). Analysis of oxysterols by electrospray tandem mass spectrometry. J Am Soc Mass Spectrom., 17(3), 341-362.)
● mono- or di-DMG esters (Jiang X., Ory D. S., Han X. (2007). Characterization of oxysterols by electrospray ionization tandem mass spectrometry after one-step derivatization with dimethylglycine. Rapid Commun Mass Spectrom, 21 (2), 141-152)
● picolinyl esters (Honda A., Yamashita K., Miyazaki H., Shirai M., Ikegami T., Xu G., Numazawa M. Hara T., Matsuzaki Y. (2008). High sensitive analysis of sterol profiles in human serum by LC-ESI-MS/MS. J. Lipid Res., 49(9), 2063-2073)

Thus, the skilled person will be aware of a number of derivatization techniques being suitable for the optimized detection of the oxysterols of the present invention. Nevertheless, such oxysterol derivatives will be encompassed by the scope of the present invention.

Furthermore, in some embodiments, the present invention provides a method of diagnosing sepsis and/or duration/severity comprising: detecting the presence or absence of one or more (e.g., 2 or more, 3 or more, 5 or more, 10 or more, etc. measured together in a multiplex or panel format) sepsis specific metabolites in a sample (e.g., a tissue (e.g., biopsy) sample, a blood sample, a serum sample, or a urine sample) from a subject; and diagnosing sepsis based on the presence of the sepsis specific metabolite.

The present invention further provides a method of screening compounds (biological samples), comprising: an animal, a tissue, a cell containing a sepsis-specific metabolite with a test compound; and detecting the level of the sepsis specific metabolite. In some embodiments, the method further comprises the step of comparing the level of the sepsis specific metabolite in the presence of the test compound or therapeutic intervention to the level of the sepsis metabolite in the absence of the sepsis specific metabolite. In some embodiments, the cell is in vitro, in a non-human mammal, or ex vivo. In some embodiments, the test compound is a small molecule or a nucleic acid (e.g., antisense nucleic acid, a siRNA, or a miRNA) or oxygen/xenon or any neuroprotective drug that inhibits the expression of an enzyme involved in the synthesis or breakdown of a sepsis specific metabolite. In some preferred embodiments, the sepsis specific metabolite groups given in Tables 2, 3 and 4.

In some embodiments, the method of use is a high throughput method. In particular, the present invention relates to a use in accordance with claims 1 to 11 and to a method disclosed in the numbered embodiments of the present description.

According to the present invention, the term "sepsis" comprises "infection associated sepsis" (e.g. caused by bacteria, fungi, viruses, parasites, and other infectious agents) and / or sepsis with non-infectious agents, such as sepsis-associated toxins, cellular components of bacteria, fungi, viruses and parasites.

A preferred method is one, wherein the biological sample is selected from the group consisting of stool; body fluids, in particular blood, liquor, cerebrospinal fluid, urine, ascitic fluid, seminal fluid, saliva, puncture fluid, cell content, tissue samples, in particular liver biopsy material; or a mixture thereof.

Advantageously, a preferred embodiment of the method according to the present invention is one, wherein said quantitative metabolomics profile is achieved by a quantitative metabolomics profile analysis method comprising the generation of intensity data for the quantitation of endogenous metabolites by mass spectrometry (MS), in particular, by high- throughput mass spectrometry, preferably by MS-technologies such as Matrix Assisted Laser Desorption/lonisation (MALDI), Electro Spray Ionization (ESI), Atmospheric Pressure Chemical Ionization (APCl),¹H-, ¹³C- and/or ³¹P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).

Furthermore, it is within the skilled expert's knowledge that in a case of need, preferably, intensity data of said metabolomics profile are normalized with a set of endogenous housekeeper metabolites by relating detected intensities of the selected endogenous sepsis predictive target metabolites to intensities of said endogenous housekeeper metabolites.

A particularly preferred method according to the present invention is one, wherein said endogenous housekeeper metabolites are selected from the group consisting of such endogeneous metabolites which show stability in accordance with statistical stability measures being selected from the group consisting of coefficient of variation (CV) of raw intensity data, standard deviation (SD) of logarithmic intensity data, stability measure (M) of geNorm - algorithm [Vandesompele et al. (2002). Accurate normalization of real-time quantitative RT-PCR data by geometric averaging of multiple internal control genes. Genome Biology, 3(7):research0034.1-0034.11.] or stability measure value (rho) of NormFinder-algorithm [Andersen et al. (2004). Normalization of Real-Time Quantitative Reverse Transcription-PCR Data: A Model-Based Variance Estimation Approach to Identify Genes Suited for Normalization, Applied to Bladder and Colon Cancer Data Sets. Cancer Research 64:5245-5250.].

Additionally, said quantitative metabolomics profile comprises the results of measuring at least one of the parameters selected from the group consisting of:
concentration, level or amount of each individual endogenous metabolite of said plurality of endogenous metabolites in said sample, qualitative and/or quantitative molecular pattern and/or molecular signature; and using and storing the obtained set of values in a database.

A panel of reference endogenous sepsis target metabolites or derivatives thereof is established by:
a) mathematically preprocessing intensity values obtained for generating the metabolomics profiles in order to reduce technical errors being inherent to the measuring procedures used to generate the metabolomics profiles;
b) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbour classifiers (K-NN), fuzzy classifiers, bagging, boosting, and naïve Bayes; and applying said selected classifier algorithm to said preprocessed data of step a);
c) said classifier algorithms of step b) being trained on at least one training data set containing preprocessed data from subjects being divided into classes according to their likelihood to develop an sepsis, in order to select a classifier function to map said preprocessed data to said likelihood;
d) applying said trained classifier algorithms of step c) to a preprocessed data set of a subject with unknown sepsis likelihood, and using the trained classifier algorithms to predict the class label of said data set in order to predict the likelihood for a subject to develop sepsis.

In the broadest sense of the present invention, said endogenous sepsis target metabolites are selected from the compounds in accordance with table 1.

For generating a metabolomics analysis profile, said plurality of endogenous sepsis (predictive) target metabolites or derivatives thereof comprises 1 to 20, in particular 1 to 15, preferably 1 to 10, preferred 1 to 5 endogenous metabolites. Furthermore, every single endogenous metabolite or voluntary groups of 2 out of the entirety of the disclosed endogenous metabolites, or 3, 4, 5, 6....19 out of the entirety of the disclosed endogenous metabolites can be used for the purpose of the present invention.

In addition to the use in accordance with claims 1 to 11, the invention relates to a method of use and to the following numbered alternative embodiments of the present invention:
1. Method for predicting the likelihood of an onset of a sepsis from a biological sample of a mammalian subject in vitro, wherein
   a. the subject's quantitative metabolomics profile comprising one or a plurality of endogenous metabolites, is detected in the biological sample by means of quantitative metabolomics analysis, and
   b. the quantitative metabolomics profile of the subject's sample is compared with a quantitative reference metabolomics profile of one or a plurality of endogenous sepsis predictive target metabolites in order to predict whether the subject is likely or unlikely to develop a sepsis; and
   c. wherein said endogenous sepsis predictive target metabolites have a molecular mass less than 1500 Da and are selected from the group consisting of:
      oxysterols, namely cholesterol, 20α-hydroxycholesterol, 22(R)-hydroxycholesterol, 22(S)-hydroxycholesterol, 24(S)-hydroxycholesterol, 25-hydroxycholesterol, 27-hydroxycholesterol, 24(R),25-epoxycholesterol, 24(S),25-epoxycholesterol, 3ß,5α,6ß-trihydroxycholestan, 7α-hydroxycholesterol, 7-Ketocholesterol, 5α,6α- epoxycholesterol 5ß,6ß-epoxycholesterol, 4ß-hydroxycholesterol, desmosterol, 7-dehydrocholesterol, cholestenone, lanosterol; 24,25-dihydrolanosterol.
2. Method according to embodiment 1, wherein the method comprises diagnosing an early sepsis and/or predicting a severe sepsis and/or predicting a septic shock.
3. Method according to embodiment 1 or 2, wherein the biological sample is selected from the group consisting of stool; body fluids, in particular blood, liquor, cerebrospinal fluid, urine, ascitic fluid, seminal fluid, saliva, puncture fluid, cell content, tissue samples, in particular liver biopsy material; or a mixture thereof.
4. Method according to anyone of embodiments 1 to 3, wherein said quantitative metabolomics profile is achieved by a quantitative metabolomics profile analysis method comprising the generation of intensity data for the quantitation of endogenous metabolites by mass spectrometry (MS), in particular, by high-throughput mass spectrometry, preferably by MS-technologies such as Matrix Assisted Laser Desorption/lonisation (MALDl), Electro Spray Ionization (ESI), Atmospheric Pressure Chemical Ionization (APCl),¹H-, ¹³C- and/or ³¹P- Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).
5. Method according to anyone of embodiments 1 to 4, wherein intensity data of said metabolomics profile are normalized with a set of endogenous housekeeper metabolites by relating detected intensities of the selected endogenous organ failure predictive target metabolites to intensities of said endogenous housekeeper metabolites.
6. Method according to embodiment 5, wherein said endogenous housekeeper metabolites are selected from the group consisting of such endogenous metabolites which show stability in accordance with statistical stability measures being selected from the group consisting of coefficient of variation (CV) of raw intensity data, standard deviation (SD) of logarithmic intensity data, stability measure (M) of geNorm - algorithm or stability measure value (rho) of NormFinder-algorithm.
7. Method according to anyone of embodiments 1 to 6, wherein said quantitative metabolomics profile comprises the results of measuring at least one of the parameters selected from the group consisting of: concentration, level or amount of each individual endogenous metabolite of said plurality of endogenous metabolites in said sample, qualitative and/or quantitative molecular pattern and/or molecular signature; and using and storing the obtained set of values in a database.
8. Method according to anyone of embodiments 1 to 7, wherein a panel of reference endogenous sepsis predictive target metabolites or derivatives thereof is established by:
   a) mathematically preprocessing intensity values obtained for generating the metabolomics profiles in order to reduce technical errors being inherent to the measuring procedures used to generate the metabolomics profiles;
   b) selecting at least one suitable classifying algorithm from the group consisting of logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbour classifiers (K-NN), fuzzy classifiers, bagging, boosting, and naïve Bayes; and applying said selected classifier algorithm to said preprocessed data of step a);
   c) said classifier algorithms of step b) being trained on at least one training data set containing preprocessed data from subjects being divided into classes according to their likelihood to develop an organ failure, in order to select a classifier function to map said preprocessed data to said likelihood;
   d) applying said trained classifier algorithms of step c) to a preprocessed data set of a subject with unknown organ failure likelihood, and using the trained classifier algorithms to predict the class label of said data set in order to predict the likelihood for a subject to develop an organ failure.
9. Method according to anyone of embodiments 1 to 8, wherein said endogenous sepsis predictive target metabolites for easier and/or more sensitive detection are detected by means of chemically modified derivatives thereof, such as acylated, silylated metabolites or a derivatization as hydrazones, in particular, Girard P hydrazones, mono- or di-dimethylglycine esters or picolinyl esters.
10. Method according to anyone of embodiments 1 to 9, wherein said endogenous target metabolites are selected from the group consisting of:
   22(R)-hydroxycholesterol, 24(S)-hydroxycholesterol, 25-hydroxycholesterol, 27-hydroxycholesterol, 24,25 epoxycholesterol, 7α-hydroxycholesterol, 4ß-hydroxycholesterol, desmosterol, 7-dehydrocholesterol, cholestenone, lanosterol, 24,25-dihydrolanosterol.
11. Method according to embodiment 10, wherein one or a plurality of said target metabolites listed in embodiment 10 is suitable for an early diagnosis of sepsis.
12. Method according to anyone of embodiments 1 to 10, wherein said endogenous target metabolites are selected from the group consisting of:
   22(R)-hydroxycholesterol, 24(S)-hydroxycholesterol, 25-hydroxycholesterol, 27-hydroxycholesterol, 3ß,5α,6ß-trihydroxycholestan, 7α-hydroxycholesterol, 7-ketocholesterol, 5ß,6ß-epoxycholesterol, 5α,6α-epoxycholesterol, 4ß-hydroxycholesterol, desmosterol, 7-dehydrocholesterol, cholestenone, lanosterol, 24,25-dihydrolanosterol.
13. Method according to embodiment 12, wherein one or a plurality of said target metabolites listed in embodiment 12 is suitable for an early diagnosis of a severe sepsis.
14. Method according to anyone of embodiments 1 to 10, wherein said endogenous target metabolites are selected from the group consisting of:
   24(S)-hydroxycholesterol, 25-hydroxycholesterol, 27-hydroxycholesterol, 7-ketocholesterol, 5ß,6ß-epoxycholesterol, 5α,6α-epoxycholesterol, 4ß-hydroxycholesterol, desmosterol, lanosterol.
15. Method according to embodiment 14, wherein one or a plurality of said target metabolites listed in embodiment 14 is suitable for determining a likelihood of an onset of a septic shock.

Furthermore, the present invention may be useful for a kit for carrying out a method for predicting the likelihood of an onset of a sepsis from a biological sample of a mammalian subject in vitro, in a biological sample, comprising:
a) calibration agents for the quantitative detection of endogenous sepsis predictive target metabolites, wherein said metabolites are selected from the group consisting of: oxysterols, namely cholesterol, 20α-hydroxycholesterol, 22(R)-hydroxycholesterol, 22(S)-hydroxycholesterol, 24(S)-hydroxycholesterol, 25-hydroxycholesterol, 27-hydroxycholesterol, 24(R),25-epoxycholesterol, 24(S),25-epoxycholesterol, 3ß**,**5α,6ß-trihydroxycholestan, 7α-hydroxycholesterol, 7-Ketocholesterol, 5α,6α-epoxycholesterol 5ß,6ß-epoxycholesterol, 4ß-hydroxycholesterol, desmosterol, 7-dehydrocholesterol, cholestenone, lanosterol; 24,25-dihydrolanosterol.
b) data base with processed data from healthy patients and patients who developed sepsis;
c) classification software for generating the quantitative metabolomics profiles achieved with said calibration agents of step a) and classifying the results based on the processed data of step b).

Data classification is the categorization of data for its most effective and efficient use. Classifiers are typically deterministic functions that map a multi-dimensional vector of biological measurements to a binary (or n-ary) outcome variable that encodes the absence or existence of a clinically-relevant class, phenotype, distinct physiological state or distinct state of disease. To achieve this various classification methods such as, but not limited to, logistic regression, (diagonal) linear or quadratic discriminant analysis (LDA, QDA, DLDA, DQDA), perceptron, shrunken centroids regularized discriminant analysis (RDA), random forests (RF), neural networks (NN), Bayesian networks, hidden Markov models, support vector machines (SVM), generalized partial least squares (GPLS), partitioning around medoids (PAM), inductive logic programming (ILP), generalized additive models, gaussian processes, regularized least square regression, self organizing maps (SOM), recursive partitioning and regression trees, K-nearest neighbor classifiers (K-NN), fuzzy classifiers, bagging, boosting, and naïve Bayes and many more can be used.

Further aspects, advantages and embodiments of the present invention will become evident by the description of examples, from the experimental sections below and by means of the drawings.

A patient with sepsis has a clinical presentation that is classified as sepsis as defined above, but is not clinically deemed to have sepsis.

As used herein, "sepsis" includes all stages of sepsis including, but not limited to, the onset of sepsis and multi organ dysfunction (MOD) , e.g. associated with the end stages of sepsis.

"Sepsis" refers to a sepsis-positive condition that is associated with a confirmed infectious process. Clinical suspicion of sepsis arises from the suspicion that the sepsis-positive condition of a sepsis patient is a result of an infectious process.

The "onset of sepsis" refers to an early stage of sepsis, i.e., prior to a stage when the clinical manifestations are sufficient to support a clinical suspicion of sepsis. Because the methods of the present invention are used to detect sepsis prior to a time that sepsis would be suspected using conventional techniques, the patient's disease status at early sepsis can only be confirmed retrospectively, when the manifestation of sepsis is more clinically obvious. The exact mechanism by which a patient acquires sepsis is not a critical aspect of the invention. The methods of the present invention can detect changes in the biomarker score independent of the origin of the sepsis. Regardless of how sepsis arises, the methods of the present invention allow for determining the status of a patient having, or suspected of having, sepsis, as classified by previously used criteria.

As used herein, the term "sepsis specific metabolite" refers to a metabolite that is differentially present or differentially concentrated in septic organisms compared to non-septic organisms. For example, in some embodiments, sepsis specific metabolites are present in septic tissues but not in non- septic tissues.

In other embodiments, sepsis -specific metabolites are absent in septic tissues but present in non-septic cells, tissues, body liquids. In still further embodiments, sepsis specific metabolites are present at different levels (e.g., higher or lower) in septic tissue/cells as compared to non-septic cells. For example, a sepsis specific metabolite may be differentially present at any level, but is generally present at a level that is increased by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, by at least 100%, by at least 110%, by at least 120%, by at least 130%, by at least 140%, by at least 150%, or more; or is generally present at a level that is decreased by at least 5%, by at least 10%, by at least 15%, by at least 20%, by at least 25%, by at least 30%, by at least 35%, by at least 40%, by at least 45%, by at least 50%, by at least 55%, by at least 60%, by at least 65%, by at least 70%, by at least 75%, by at least 80%, by at least 85%, by at least 90%, by at least 95%, or by 100% (i.e., absent).

A sepsis -specific metabolite is preferably differentially present at a level that is statistically significant (e.g., an (adjusted) p-value less than 0.05 as determined using either Analysis of Variance, Welch's t-test or its non parametric equivalent versions). Exemplary sepsis -specific metabolites are described in the detailed description and experimental sections below.

The term "sample" in the present specification and claims is used in its broadest sense. On the one hand it is meant to include a specimen or culture. On the other hand, it is meant to include both biological and environmental samples. A sample may include a specimen of synthetic origin.

Biological samples may be animal, including human, fluid, solid (e.g., stool) or tissue, such biological samples may be obtained from all of the various families of domestic animals, as well as feral or wild animals, including, but not limited to, such animals as ungulates, bear, fish, rodents, etc. A biological sample may contain any biological material suitable for detecting the desired biomarkers, and may comprise cellular and/or non-cellular material from a subject. The sample can be isolated from any suitable biological tissue or fluid such as, for example, tissue, blood, blood plasma, urine, or cerebral spinal fluid (CSF).

A "reference level" of a metabolite means a level of the metabolite that is indicative of a particular disease state, phenotype, or lack thereof, as well as combinations of disease states, phenotypes, or lack thereof. A "positive" reference level of a metabolite means a level that is indicative of a particular disease state or phenotype. A "negative" reference level of a metabolite means a level that is indicative of a lack of a particular disease state or phenotype. For example, a "sepsis -positive reference level" of a metabolite means a level of a metabolite that is indicative of a positive diagnosis of sepsis in a subject, and an "sepsis -negative reference level" of a metabolite means a level of a metabolite that is indicative of a negative diagnosis of sepsis in a subject. A "reference level" of a metabolite may be an absolute or relative amount or concentration of the metabolite, a presence or absence of the metabolite, a range of amount or concentration of the metabolite, a minimum and/or maximum amount or concentration of the metabolite, a mean amount or concentration of the metabolite, and/or a median amount or concentration of the metabolite; and, in addition, "reference levels" of combinations of metabolites may also be ratios of absolute or relative amounts or concentrations of two or more metabolites with respect to each other or a composed value / score obtained by classification.

Appropriate positive and negative reference levels of metabolites for a particular disease state, phenotype, or lack thereof may be determined by measuring levels of desired metabolites in one or more appropriate subjects, and such reference levels may be tailored to specific populations of subjects (e.g., a reference level may be age-matched so that comparisons may be made between metabolite levels in samples from subjects of a certain age and reference levels for a particular disease state, phenotype, or lack thereof in a certain age group). Such reference levels may also be tailored to specific techniques that are used to measure levels of metabolites in biological samples (e.g., LC-MS, GC-MS, etc.), where the levels of metabolites may differ based on the specific technique that is used.

As used herein, the term "cell" refers to any eukaryotic or prokaryotic cell (e.g., bacterial cells such as E. coli, yeast cells, mammalian cells, avian cells, amphibian cells, plant cells, fish cells, and insect cells), whether located in vitro or in vivo.

As used herein, the term "processor" refers to a device that performs a set of steps according to a program (e.g., a digital computer). Processors, for example, include Central Processing Units ("CPUs"), electronic devices, or systems for receiving, transmitting, storing and/or manipulating data under programmed control.

As used herein, the term "memory device," or "computer memory" refers to any data storage device that is readable by a computer, including, but not limited to, random access memory, hard disks, magnetic (floppy) disks, compact discs, DVDs, magnetic tape, flash memory, and the like.

"Mass Spectrometry" (MS) is a technique for measuring and analyzing molecules that involves fragmenting a target molecule, then analyzing the fragments, based on their mass/charge ratios, to produce a mass spectrum that serves as a "molecular fingerprint". Determining the mass/charge ratio of an object is done through means of determining the wavelengths at which electromagnetic energy is absorbed by that object. There are several commonly used methods to determine the mass to charge ration of an ion, some measuring the interaction of the ion trajectory with electromagnetic waves, others measuring the time an ion takes to travel a given distance, or a combination of both. The data from these fragment mass measurements can be searched against databases to obtain definitive identifications of target molecules. Mass spectrometry is also widely used in other areas of chemistry, like petrochemistry or pharmaceutical quality control, among many others.

As used here, the term "metabolite" denotes endogenous organic compounds of a cell, an organism, a tissue or being present in body liquids and in extracts obtained from the aforementioned sources with a molecular weight typically below 1500 Dalton. Typical examples of metabolites are carbohydrates, lipids, phospholipids, sphingolipids and sphingophospholipids, amino acids, cholesterol, steroid hormones and oxidized sterols and other compounds such as collected in the Human Metabolite database [Wishart DS et al., HMDB: the Human Metabolome Database. Nucleic Acids Res. 2007 Jan;35 (Database issue):D521-6(see http://www.hmdb.ca/)*]* and other databases and literature. This includes any substance produced by metabolism or by a metabolic process and any substance involved in metabolism.

"Metabolomics" as understood within the scope of the present invention designates the comprehensive quantitative measurement of several (2-thousands) metabolites by, but not limited to, methods such as mass spectroscopy, coupling of liquid chromatography, gas chromatography and other separation methods chromatography with mass spectroscopy.

The term "separation" refers to separating a complex mixture into its component proteins or metabolites. Common laboratory separation techniques include gel electrophoresis and chromatography.

The term "capillary electrophoresis" refers to an automated analytical technique that separates molecules in a solution by applying voltage across buffer-filled capillaries. Capillary electrophoresis is generally used for separating ions, which move at different speeds when the voltage is applied, depending upon the size and charge of the ions. The solutes (ions) are seen as peaks as they pass through a detector and the area of each peak is proportional to the concentration of ions in the solute, which allows quantitative determinations of the ions.

The term "chromatography" refers to a physical method of separation in which the components to be separated are distributed between two phases, one of which is stationary (stationary phase) while the other (the mobile phase) moves in a definite direction. Chromatographic output data may be used for manipulation by the present invention.

An "ion" is a charged object formed by adding electrons to or removing electrons from an atom.

A "mass spectrum" is a plot of data produced by a mass spectrometer, typically containing m/z values on x-axis and intensity values on y-axis.

A "peak" is a point on a mass spectrum with a relatively high y-value.

The term "m/z" refers to the dimensionless quantity formed by dividing the mass number of an ion by its charge number. It has long been called the "mass-to-charge" ratio.

The term "metabolism" refers to the chemical changes that occur within the tissues of an organism, including "anabolism" and "catabolism". Anabolism refers to biosynthesis or the buildup of molecules and catabolism refers to the breakdown of molecules.

As used herein, the term "post-surgical tissue" refers to tissue that has been removed from a subject during a surgical procedure. Examples include, but are not limited to, biopsy samples, excised organs, and excised portions of organs.

As used herein, the terms "detect", "detecting", or "detection" may describe either the general act of discovering or discerning or the specific observation of a detectably labelled composition.

As used herein, the term "clinical failure" refers to a negative outcome following sepsis treatment.

A biomarker in this context is a characteristic, comprising data of at least one metabolite that is measured and evaluated as an indicator of biologic processes, pathogenic processes, or responses to a therapeutic intervention associated with sepsis or related to sepsis treatment. A combined biomarker as used here may be selected from at least two small endogenous molecules and metabolites.

### DETAILED DESCRIPTION OF THE INVENTION

The present invention relates to markers of sepsis and its duration/severity as well of the effect of therapeutic interventions. In particular embodiments, the present invention provides metabolites that are differentially present in sepsis. Experiments conducted during the course of development of embodiments of the present invention identified a series of metabolites as being differentially present in tables 1 to 4 provide metabolites present in plasma serum or other body liquids. The disclosed markers find use as diagnostic and therapeutic targets.

### Diagnostic Applications

In some embodiments, the present invention provides methods and compositions for diagnosing sepsis, including but not limited to, characterizing risk of sepsis, stage of sepsis, duration and severity etc. based on the presence of sepsis specific metabolites or their derivatives, precursors, metabolites, etc. Exemplary diagnostic methods are described below.

Thus, for example, a method of diagnosing (or aiding in diagnosing) whether a subject has sepsis comprises (1) detecting the presence or absence or a differential level of one or more sepsis specific metabolites selected from table 1 and b) diagnosing sepsis based on the presence, absence or differential level of the sepsis specific metabolite. When such a method is used to aid in the diagnosis of sepsis, the results of the method may be used along with other methods (or the results thereof) useful in the clinical determination of whether a subject has sepsis.

Any mammalian sample suspected of containing sepsis specific metabolites is tested according to the methods described herein. By way of non-limiting examples, the sample may be tissue (e.g., a biopsy sample or post-surgical tissue), blood, urine, or a fraction thereof (e.g., plasma, serum, urine supernatant, urine cell pellet).

In some embodiments, the patient sample undergoes preliminary processing designed to isolate or enrich the sample for sepsis specific metabolites or cells that contain sepsis specific metabolites. A variety of techniques known to those of ordinary skill in the art may be used for this purpose, including but not limited to: centrifugation, immunocapture, and cell lysis.

Metabolites may be detected using any suitable method including, but not limited to, liquid and gas phase chromatography, alone or coupled to mass spectrometry (See e.g., experimental section below), NMR, immunoassays, chemical assays, spectroscopy and the like. In some embodiments, commercial systems for chromatography and NMR analysis are utilized.

In other embodiments, metabolites (i.e. biomarkers and derivatives thereof) are detected using optical imaging techniques such as magnetic resonance spectroscopy (MRS), magnetic resonance imaging (MRI), CAT scans, ultra sound, MS-based tissue imaging or X-ray detection methods (e.g., energy dispersive x-ray fluorescence detection).

Any suitable method may be used to analyze the biological sample in order to determine the presence, absence or level(s) of the one or more metabolites in the sample. Suitable methods include chromatography (e.g., HPLC, gas chromatography, liquid chromatography), mass spectrometry (e.g., MS, MS-MS), enzyme-linked immunosorbent assay (ELISA), antibody linkage, other immunochemical techniques, biochemical or enzymatic reactions or assays, and combinations thereof. Further, the level(s) of the one or more metabolites may be measured indirectly, for example, by using an assay that measures the level of a compound (or compounds) that correlates with the level of the biomarker(s) that are desired to be measured.

The levels of one or more of the recited metabolites may be determined in the methods of the present invention. For example, the level(s) of one metabolites, two or more metabolites, three or more metabolites, four or more metabolites, five or more metabolites, six or more metabolites, seven or more metabolites, eight or more metabolites, nine or more metabolites, ten or more metabolites, etc., including a combination of some or all of the metabolites including, but not limited to those listed in table 2, may be determined and used in such methods.

Determining levels of combinations of the metabolites may allow greater sensitivity and specificity in the methods, such as diagnosing sepsis and aiding in the diagnosis of sepsis, and may allow better differentiation or characterization of sepsis from other disorders or other sepsis that may have similar or overlapping metabolites to sepsis (as compared to a subject not having sepsis). For example, ratios of the levels of certain metabolites in biological samples may allow greater sensitivity and specificity in diagnosing sepsis and aiding in the diagnosis of sepsis and allow better differentiation or characterization of sepsis from other sepsis or other disorders of the that may have similar or overlapping metabolites to sepsis (as compared to a subject not having sepsis).

### Data Analysis

In some embodiments, a computer-based analysis program is used to translate the raw data generated by the detection assay (e.g., the presence, absence, or amount of a sepsis specific metabolite) into data of predictive value for a clinician. The clinician can access the predictive data using any suitable means. Thus, in some embodiments, the present invention provides the further benefit that the clinician, who is not likely to be trained in metabolite analysis, need not understand the raw data. The data is presented directly to the clinician in its most useful form. The clinician is then able to immediately utilize the information in order to optimize the care of the subject.

The present invention contemplates any method capable of receiving, processing, and transmitting the information to and from laboratories conducting the assays, information provides, medical personal, and subjects. For example, in some embodiments of the present invention, a sample (e.g., a biopsy or a blood, urine or serum sample) is obtained from a subject and submitted to a profiling service (e.g., clinical lab at a medical facility, etc.), located in any part of the world (e.g., in a country different than the country where the subject resides or where the information is ultimately used) to generate raw data. Where the sample comprises a tissue or other biological sample, the subject may visit a medical center to have the sample obtained and sent to the profiling center, or subjects may collect the sample themselves (e.g., a plasma sample) and directly send it to a profiling center. Where the sample comprises previously determined biological information, the information may be directly sent to the profiling service by the subject (e.g., an information card containing the information may be scanned by a computer and the data transmitted to a computer of the profiling center using an electronic communication systems). Once received by the profiling service, the sample is processed and a profile (i.e., metabolic profile), specific for the diagnostic or prognostic information desired for the subject is produced.

The profile data is then prepared in a format suitable for interpretation by a treating clinician. For example, rather than providing raw data, the prepared format may represent a diagnosis or risk assessment (e.g., likelihood of sepsis being present) for the subject, along with recommendations for particular treatment options. The data may be displayed to the clinician by any suitable method. For example, in some embodiments, the profiling service generates a report that can be printed for the clinician (e.g., at the point of care) or displayed to the clinician on a computer monitor.

In some embodiments, the information is first analyzed at the point of care or at a regional facility. The raw data is then sent to a central processing facility for further analysis and/or to convert the raw data to information useful for a clinician or patient. The central processing facility provides the advantage of privacy (all data is stored in a central facility with uniform security protocols), speed, and uniformity of data analysis. The central processing facility can then control the fate of the data following treatment of the subject. For example, using an electronic communication system, the central facility can provide data to the clinician, the subject, or researchers.

In some embodiments, the subject is able to directly access the data using the electronic communication system. The subject may chose further intervention or counselling based on the results. In some embodiments, the data is used for research use. For example, the data may be used to further optimize the inclusion or elimination of markers as useful indicators of a particular condition or stage of disease.

When the amount(s) or level(s) of the one or more metabolites in the sample are determined, the amount(s) or level(s) may be compared to sepsis metabolite-reference levels, such as - sepsis -positive and/or sepsis -negative reference levels to aid in diagnosing or to diagnose whether the subject has sepsis. Levels of the one or more metabolites in a sample corresponding to the sepsis -positive reference levels (e.g., levels that are the same as the reference levels, substantially the same as the reference levels, above and/or below the minimum and/or maximum of the reference levels, and/or within the range of the reference levels) are indicative of a diagnosis of sepsis in the subject. Levels of the one or more metabolites in a sample corresponding to the sepsis -negative reference levels (e.g., levels that are the same as the reference levels, substantially the same as the reference levels, above and/or below the minimum and/or maximum of the reference levels, and/or within the range of the reference levels) are indicative of a diagnosis of no sepsis in the subject. In addition, levels of the one or more metabolites that are differentially present (especially at a level that is statistically significant) in the sample as compared to sepsis-negative reference levels are indicative of a diagnosis of sepsis in the subject. Levels of the one or more metabolites that are differentially present (especially at a level that is statistically significant) in the sample as compared to sepsis -positive reference levels are indicative of a diagnosis of no sepsis in the subject.

The level(s) of the one or more metabolites may be compared to sepsis -positive and/or sepsis -negative reference levels using various techniques, including a simple comparison (e.g., a manual comparison) of the level(s) of the one or more metabolites in the biological sample to sepsis -positive and/or sepsis -negative reference levels. The level(s) of the one or more metabolites in the biological sample may also be compared to sepsis and/or sepsis -negative reference levels using one or more statistical analyses (e.g., t-test, Welch's t-test, Wilcoxon's rank sum test, random forests, support vector machines, linear discriminant analysis, k nearest neighbours).

Compositions for use (e.g., sufficient for, necessary for, or useful for) in the diagnostic methods of some embodiments of the present invention include reagents for detecting the presence or absence of sepsis specific metabolites. Any of these compositions, alone or in combination with other compositions of the present invention, may be provided in the form of a kit. Kits may further comprise appropriate controls and/or detection reagents.

Embodiments of the present invention provide for multiplex or panel assays that simultaneously detect one or more of the markers of the present invention depicted in tables 1 to 3, alone or in combination with additional sepsis markers known in the art. For example, in some embodiments, panel or combination assays are provided that detected 2 or more, 3 or more, 4 or more, 5 or more, 6 or more, 7 or more, 8 or more, 9 or more, 10 or more, 15 or more, or 20 or more, 30 or more, 40 or more markers in a single assay. In some embodiments, assays are automated or high throughput.

A preferred embodiment of the present invention is the use of markers listed in tables 2 and 3 for prediction/diagnosis of sepsis and its duration/severity where said mammalian subject is a human being, said biological sample blood and/or blood cells.

In some embodiments, additional sepsis markers are included in multiplex or panel assays. Markers are selected for their predictive value alone or in combination with the metabolic markers described herein.

### Therapeutic Methods

In some embodiments, the present invention provides therapeutic methods (e.g., that target the sepsis specific metabolites described herein). In some embodiments, the therapeutic methods target enzymes or pathway components of the sepsis specific metabolites described herein.

For example, in some embodiments, the present invention provides compounds that target the sepsis specific metabolites of the present invention. The compounds may decrease the level of sepsis specific metabolite by, for example, interfering with synthesis of the sepsis specific metabolite (e.g., by blocking transcription or translation of an enzyme involved in the synthesis of a metabolite, by inactivating an enzyme involved in the synthesis of a metabolite (e.g., by post translational modification or binding to an irreversible inhibitor), or by otherwise inhibiting the activity of an enzyme involved in the synthesis of a metabolite) or a precursor or metabolite thereof, by binding to and inhibiting the function of the sepsis specific metabolite, by binding to the target of the sepsis specific metabolite (e.g., competitive or non competitive inhibitor), or by increasing the rate of break down or clearance of the metabolite.

The compounds may increase the level of sepsis specific metabolite by, for example, inhibiting the break down or clearance of the sepsis specific metabolite (e.g., by inhibiting an enzyme involved in the breakdown of the metabolite), by increasing the level of a precursor of the sepsis specific metabolite, or by increasing the affinity of the metabolite for its target.

Dosing is dependent on severity and responsiveness of the disease state to be treated, with the course of treatment lasting from several days to several months, or until a cure is effected or a diminution of the disease state is achieved. Optimal dosing schedules can be calculated from measurements of drug accumulation in the body of the patient. The administering physician can easily determine optimum dosages, dosing methodologies and repetition rates.

In some embodiments, the present invention provides drug screening assays (e.g., to screen for anti - sepsis drugs). The screening methods of the present invention utilize sepsis specific metabolites described herein. As described above, in some embodiments, test compounds are small molecules, nucleic acids, or antibodies. In some embodiments, test compounds target sepsis specific metabolites directly. In other embodiments, they target enzymes involved in metabolic pathways of sepsis specific metabolites.

### EXPERIMENTAL

The following examples are provided in order to demonstrate and further illustrate certain preferred embodiments and aspects of the present invention and are not to be construed as limiting the scope thereof.

### General Analytics:

Sample preparation and metabolomic analyses were performed at BIOCRATES life sciences AG, Innsbruck, Austria. We used a multi-parametric, highly robust, sensitive and high-throughput targeted metabolomic platform consisting of flow injection analysis (FIA)-MS/MS and LC-MS/MS methods for the simultaneous quantification of a broad range of endogenous intermediates namely from the panel disclosed in table 1. All procedures (sample handling, analytics) were performed by co-workers blinded to the groups.

### Plasma homogenization

Plasma samples were prepared by standard procedures and stored at (-70°C). To enable analysis of all samples simultaneously within one batch, samples were thawed on ice (1 h) on the day of analysis and centrifuged at 18000 *g* at 2°C for 5 min. All tubes were prepared with 0.001 % BHT (butylated hydroxytoluene; Sigma-Aldrich, Vienna, Austria) to prevent artificial formation of prostaglandins caused by autooxidation .

### Determination of Oxysterols

Oxysterols are determined after extraction and saponification by HPLC-Tandem mass spectrometer (HPLC-API-MS/MS) in positive detection mode using Multiple Reaction Mode (MRM).

Samples (20 µL), calibrators and internal standard mixture were placed into a capture plate and were protein precipitated in the first step by means of addition of 200 µL acetonitrile and centrifugation. 180 µL of the appropriate supernatants were transferred on a new filter plate with 7 mm filter spots, dried down, hydrolysed with 0.35 M KOH in 95 % Ethanol and after washing steps extracted with 100 µL aqueous MeOH. An aliquot of the extracted sample is injected onto the HPLC-MS/MS system. Chromatographic separation and detection is performed by using a Zorbax Eclipse XDB C18, 150 x 2.0 mm, 3.5 µm HPLC-Column at a flow rate of 0.3 mL/min followed by electrospray ionization on the AP14000/QTRAP4000 tandem mass spectrometer. For the quantitation the Analyst Quantitation software from Applied Bioystems was used.

**Table 1 summarizes analyzed oxysterol metabolites and respective abbreviations**

| **Name** | **Common Name** | **CAS Registry Number** |
|---|---|---|
| 20aOHC | 20α-Hydroxycholesterol | 516-72-3 |
| 22ROHC | 22-R-Hydroxycholesterol | 17954-98-2 |
| 22SOHC | 22S-Hydroxycholesterol | 22348-64-7 |
| 24,25,EPC | 24,25-Epoxycholesterol | 68138-65-8 |
| 24,25EC | 24,25-Epoxycholesterol | 68138-65-8 |
| 24DHLan | 24-Dihydrolanosterol | 79-62-9 |
| 24SOHC | 24-S-Hydroxycholesterol | 474-73-7 |
| 250HC | 25-Hydroxycholesterol | 2140-46-7 |
| 270HC | 27-Hydroxycholesterol | 20380-11-4 |
| 3B,5a,6BTHC | 3ß,5α,6ß-Trihydroxycholestan | 1253-84-5 |
| 4BOHC | 4ß-Hydroxycholesterol | 17320-10-4 |
| 5a,6a,EC | 5α,6α-Epoxycholesterol | 2953-38-0 |
| 5a,6a,EPC | 5α,6α-Epoxycholesterol | 2953-38-0 |
| 5B,6B,EC | 5ß,6ß-Epoxycholesterol | 4025-59-6 |
| 5B,6B,EPC | 5ß,6ß-Epoxycholesterol | 4025-59-6 |
| 7aOHC | 7α-Hydroxycholesterol | 566-26-7 |
| 7DHC | 7-Dehydrocholesterol (Vitamin D3) | 434-16-2 |
| 7KC | 7-Ketocholesterol | 566-28-9 |
| Cholestenone | Cholestenone | 601-54-7 |
| Cholesterol | | 57-88-5 |
| Desmosterol | Desmosterol | 313-04-2 |
| Lanosterol | Lanosterol | 79-63-0 |

### Detailed Examples

### 1. Sepsis

The two study groups (50 sepsis cases and 20 controls) were well balanced with regard to demographic characteristics (age and sex-matched). Inclusion criteria were clinical evidence of infection, evidence of a systemic response to infection. Notable exclusion criteria included underlying disease with a poor prognosis, a life expectancy of less than 24 hours, immunosuppression, The sepsis group comprised various types and infecting organisms (e.g. pseudomonas aeruginosa) and sites of infection (lung, pneumonia, peritoneum and others). The control group comprised hospitalized patients and patients undergoing bypass surgery (samples drawn >48 after operation, no clinical signs of sepsis and infection)

### Statistical Analysis

All statistical calculations have been performed using the statistics software R (R: A Language and Environment for Statistical Computing, R Development Core Team, R Foundation for Statistical Computing,Vienna, Austria, 2009, ISBN 3-900051-07-0).

All analytes that were detected in at least 15% of the samples were selected for further analyses. The metabolic data is left censored due to thresholding of the mass spectrometer data resulting in non detected peak/signals. By a combination of metabolic pathway dynamism, complex sample molecular interaction and overall efficiency of the analytical protocol, replacement of missing data by means of a multivariate algorithm is preferred to a naive imputation by a pre-specified value like for instance zero. Hence, missing metabolite concentrations are replaced by the average value of the 6 closest samples to the one where the measurement is missing (impute: Imputation for microarray data, Hastie T., Tibshirani R., Narasimhan B. and Chu G., R package version 1.14.0). At the exception of fold change (FC) determination, all statistical analyses are performed on preprocessed ― that is, log transformed ― data.

The ImFit function in the package limma (Limma: linear models for microarray data, Smyth G.K. In: Bioinformatics and Computational Biology Solutions using R and Bioconductor, Springer, New York, pp 397-420, R package version 2.16.5) is used to compute the moderated statistics for pairwise comparisons between measurements from control samples and samples with sepsis. Resulting p values are adjusted by the method described in Benjamini and Hochberg (Benjamini Y. and Hochberg Y., Controlling the false discovery rate: a practical and powerful approach to multiple testing, Journal of the Royal Statistical Society Series B, 1995, 57, 289-300) leading to so-called q values (adjusted p values).

Sensitivity/specificity properties of a classifier comprising one analyte or a combination of analytes are summarised in terms of Area Under the Receiver Operating Characteristic Curve (AUC). The function colAUC (caTools: Tools: moving window statistics, GIF, Base64, ROC AUC, etc., Tuszynski J., 2008, R package version 1.9) is used to compute AUC values. The results are given in Table 2:

**Table 2: AUC; (area under the curve), FC(fold change), p and q values tor cases vs. controls. The positive FC %-values are calculated as 100*(mean value of cases/mean value of controls - 1) and the negative FC %-values as -100*(mean value of controls/mean value of cases - 1).**

| **Name** | **p value** | **q value** | **AUC** | **FC [%]** |
|---|---|---|---|---|
| 22ROHC | 7,78E-003 | 1,19E-002 | 0,70 | -54,0 |
| 24SOHC | 2,64E-005 | 7,02E-005 | 0,67 | -127,7 |
| 250HC | 1,50E-002 | 2,19E-002 | 0,69 | -95,7 |
| 270HC | 5,85E-002 | 7,48E-002 | 0,64 | -34,2 |
| 24,25,EPC | 1,76E-002 | 2,54E-002 | 0,67 | -24,0 |
| 7aOHC | 2,29E-008 | 1,36E-007 | 0,90 | 128,3 |
| 4BOHC | 7,96E-005 | 1,96E-004 | 0,74 | 102,8 |
| Desmosterol | 4,69E-009 | 2,96E-008 | 0,89 | -201,7 |
| 7DHC | 4,17E-004 | 8,10E-004 | 0,69 | -174,7 |
| Cholestenone | 4,42E-003 | 7,32E-003 | 0,68 | 98,3 |
| Lanosterol | 9,86E-001 | 9,90E-001 | 0,52 | -40,0 |
| 24DHLan | 4,16E-003 | 7,00E-003 | 0,77 | 210,0 |

### 2. Severe Sepsis

The two groups (17 sepsis cases and 21 controls) were balanced with regard to demographic characteristics (age and sex-matched). Inclusion criteria were evidence of a systemic response to infection, and or the onset of shock within the previous 72 hours (as defined by a systolic blood pressure of <90 mm Hg despite adequate fluid replacement or a need for vasopressors for at least 1 hour) and hypoperfusion or organ dysfunction attributable to severe sepsis. Notable exclusion criteria included underlying disease with a poor prognosis, a life expectancy of less than 24 hours, immunosuppression. The control group comprised subjects with various types and infecting organisms (e.g. pseudomonas aeruginosa) and sites of infection (lung, pneumonia, peritoneum and others) as well as subjects with indicators for early sepsis (within 24 h after first diagnosis clinical evidence of infection and / or evidence of a systemic response to infection). The control group also included hospitalized patients and patients undergoing bypass surgery (samples drawn >48 after operation, no clinical signs of sepsis and infection).

### Statistical Analysis

All statistical calculations have been performed using the statistics software R (R: A Language and Environment for Statistical Computing, R Development Core Team, R Foundation for Statistical Computing,Vienna, Austria, 2009, ISBN 3-900051-07-0).

All analytes that were detected in at least 15% of the samples were selected for further analyses. The metabolic data is left censored due to thresholding of the mass spectrometer data resulting in non detected peak/signals. By a combination of metabolic pathway dynamism, complex sample molecular interaction and overall efficiency of the analytical protocol, replacement of missing data by means of a multivariate algorithm is preferred to a naive imputation by a pre-specified value like for instance zero. Hence, missing metabolite concentrations are replaced by the average value of the 6 closest samples to the one where the measurement is missing (impute: Imputation for microarray data, Hastie T., Tibshirani R., Narasimhan B. and Chu G., R package version 1.14.0). At the exception of fold change (FC) determination, all statistical analyses are performed on preprocessed ―that is, log transformed ― data.

The ImFit function in the package limma (Limma: linear models for microarray data, Smyth G.K. In: Bioinformatics and Computational Biology Solutions using R and Bioconductor, Springer, New York, pp 397-420, R package version 2.16.5) is used to compute the moderated statistics for pairwise comparisons between measurements from control samples and samples with severe sepsis. Resulting p values are adjusted by the method described in Benjamini and Hochberg (Benjamini Y. and Hochberg Y., Controlling the false discovery rate: a practical and powerful approach to multiple testing, Journal of the Royal Statistical Society Series B, 1995, 57, 289-300) leading to so-called q values (adjusted p values).

Sensitivity/specificity properties of a classifier comprising one analyte or a combination of analytes are summarised in terms of Area Under the Receiver Operating Characteristic Curve (AUC). The function colAUC (caTools: Tools: moving window statistics, GIF, Base64, ROC AUC, etc., Tuszynski J., 2008, R package version 1.9) is used to compute AUC values. The results are given in Table 3.

**Table 3: AUC (area under the curve), FC (fold change), p and q values for cases vs. controls. The positive FC %-values are calculated as 100*(mean value of cases/mean value of controls - 1) and the negative FC %-values as -100*(mean value of controls/mean value of cases - 1).**

| **Analyte** | **p value** | **q value** | **AUC** | **FC [%]** |
|---|---|---|---|---|
| 22ROHC | 9,42E-002 | 1,57E-001 | 0,57 | -15,1 |
| 24SOHC | 5,00E-006 | 1,50E-005 | 0,91 | -79,3 |
| 250HC | 4,15E-006 | 1,50E-005 | 0,92 | -101,0 |
| 270HC | 4,54E-003 | 1,13E-002 | 0,78 | -42,6 |
| 3B,5a,6BTHC | 3,47E-001 | 4,89E-001 | 0,64 | 164,8 |
| 7aOHC | 4,95E-008 | 3,71E-007 | 0,89 | -311,4 |
| 7KC | 6,80E-001 | 7,29E-001 | 0,52 | 33,4 |
| 5B,6B,EPC | 3,91 E-001 | 4,89E-001 | 0,50 | -54,7 |
| 5a,6a,EPC | 3,66E-001 | 4,89E-001 | 0,63 | 171,6 |
| 4BOHC | 6,13E-011 | 9,20E-010 | 0,91 | -165,8 |
| Desmosterol | 9,45E-001 | 9,45E-001 | 0,51 | -13,2 |
| 7DHC | 3,11E-002 | 5,82E-002 | 0,69 | 47,4 |
| Cholestenone | 6,15E-001 | 7,10E-001 | 0,71 | 19,8 |
| Lanosterol | 4,22E-006 | 1,50E-005 | 0,91 | 123,7 |
| 24DHLan | 1,63E-002 | 3,49E-002 | 0,55 | 95,7 |

### 3. Septic Shock

In the septic shock group (45 cases) the following two criteria were met:
1. Evidence of infection, through a positive blood culture.
2. Refractory hypotension - hypotension despite adequate fluid resuscitation and cardiac output.
   o In adults it is defined as a systolic blood pressure < 90 mmHg, or a mean arterial pressure < 60 mmHg, without the requirement for inotropic support, or a reduction of 40 mmHg in the systolic blood pressure from baseline.
   o In children it is BP < 2 standard deviations of the normal blood pressure.

In addition to the two criteria above, two or more of the following were present:
● Tachypnea (high respiratory rate) > 20 breaths per minute or, on blood gas, a partial pressure of CO₂ of less than 32 mmHg.
● White blood cell count < 4000 cells/mm³ or > 12000 cells/mm³

The control group comprised subjects with various types and infecting organisms (e.g. pseudomonas aeruginosa)and sites of infection (lung, pneumonia, peritoneum and others) as well as subjects with indicators for early sepsis (within 24 h after first diagnosis clinical evidence of infection and / or evidence of a systemic response to infection). The control group (21 cases) also included hospitalized patients and patients undergoing bypass surgery (samples drawn >48 after operation, no clinical signs of sepsis and infection).

### Statistical Analysis

All statistical calculations have been performed using the statistics software R (R: A Language and Environment for Statistical Computing, R Development Core Team, R Foundation for Statistical Computing,Vienna, Austria, 2009, ISBN 3-900051-07-0).

All analytes that were detected in at least 15% of the samples were selected for further analyses. The metabolic data is left censored due to thresholding of the mass spectrometer data resulting in non detected peak/signals. By a combination of metabolic pathway dynamism, complex sample molecular interaction and overall efficiency of the analytical protocol, replacement of missing data by means of a multivariate algorithm is preferred to a naive imputation by a pre-specified value like for instance zero. Hence, missing metabolite concentrations are replaced by the average value of the 6 closest samples to the one where the measurement is missing (impute: Imputation for microarray data, Hastie T., Tibshirani R., Narasimhan B. and Chu G., R package version 1.14.0). At the exception of fold change (FC) determination, all statistical analyses are performed on preprocessed ― that is, log transformed ― data.

The ImFit function in the package limma (Limma: linear models for microarray data, Smyth G.K. In: Bioinformatics and Computational Biology Solutions using R and Bioconductor, Springer, New York, pp 397-420, R package version 2.16.5) is used to compute the moderated statistics for pairwise comparisons between measurements from control samples and samples with septic shock. Resulting p values are adjusted by the method described in Benjamini and Hochberg (Benjamini Y. and Hochberg Y., Controlling the false discovery rate: a practical and powerful approach to multiple testing, Journal of the Royal Statistical Society Series B, 1995, 57, 289-300) leading to so-called q values (adjusted p values).

Sensitivity/specificity properties of a classifier comprising one analyte or a combination of analytes are summarised in terms of Area Under the Receiver Operating Characteristic Curve (AUC). The function colAUC (caTools: Tools: moving window statistics, GIF, Base64, ROC AUC, etc., Tuszynski J., 2008, R package version 1.9) is used to compute AUC values. The results are given in Table 4.

**Table 4: AUC (area under the curve), FC (fold change), p and q values for cases vs. controls. The positive FC %-values are calculated as 100*(mean value of cases/mean value of controls - 1) and the negative FC %-values as -100*(mean value of controls/mean value of cases - 1).**

| **Name** | **p value** | **q value** | **AUC** | **FC [%]** |
|---|---|---|---|---|
| 24SOHC | 3,28E-003 | 8,48E-003 | 0,71 | -36,7 |
| 250HC | 1,16E-008 | 1,00E-007 | 0,89 | -126,8 |
| 270HC | 7,01 E-004 | 2,08E-003 | 0,79 | -62,8 |
| 7KC | 9,38E-042 | 1,00E-015 | 0,88 | -2210,7 |
| 5B,6B,EC | 9,23E-020 | 1,00E-015 | 0,86 | -1670,3 |
| 5a,6a,EC | 3,09E-011 | 4,24E-010 | 0,83 | -294,7 |
| 4BOHC | 9,07E-028 | 1,00E-015 | 0,93 | -1029,6 |
| Desmosterol | 7,16E-001 | 7,79E-001 | 0,65 | -55,0 |
| Lanosterol | 1,19E-003 | 3,35E-003 | 0,51 | 107,2 |

## Claims

1. Use of one or a plurality of endogenous target metabolites for predicting a likelihood of an onset of a sepsis from a biological sample of a mammalian subject in vitro, wherein
said endogenous target metabolites have a molecular mass less than 1500 Da and are selected from the group consisting of:
oxysterols, namely cholesterol, 20α-hydroxycholesterol, 22(R)-hydroxycholesterol, 22(S)-hydroxycholesterol, 24(S)-hydroxycholesterol, 25-hydroxycholesterol, 27-hydroxycholesterol, 24(R),25-epoxycholesterol, 24(S),25-epoxycholesterol, 3ß,5α,6ß-trihydroxycholestan, 7α-hydroxycholesterol, 7-Ketocholesterol, 5α,6α- epoxycholesterol 5ß,6ß-epoxycholesterol, 4ß-hydroxycholesterol, desmosterol, 7-dehydrocholesterol, cholestenone, lanosterol; 24,25-dihydrolanosterol.

2. Use according to claim 1, **characterized in that** the subject's quantitative metabolomics profile comprising one or a plurality of said endogenous metabolites, is detected in the biological sample by means of quantitative metabolomics analysis, and
the quantitative metabolomics profile of the subject's sample is compared with a quantitative reference metabolomics profile of a plurality of endogenous sepsis target metabolites in order to predict whether the subject is likely or unlikely to develop a sepsis.

3. Use according to claim 1 or 2, wherein said endogenous target metabolites for easier and/or more sensitive detection are detected by means of chemically modified derivatives thereof, such as acylated, silylated derivatives, or as hydrazones, in particular, Girard P hydrazones, mono- or di-dimethylglycine esters or picolinyl esters of said target metabolites.

4. Use according to anyone of claims 1 to 3, wherein said endogenous target metabolites are selected from the group consisting of:
22(R)-hydroxycholesterol, 24(S)-hydroxycholesterol, 25-hydroxycholesterol, 27-hydroxycholesterol, 24,25 epoxycholesterol, 7α-hydroxycholesterol, 4ß-hydroxycholesterol, desmosterol, 7-dehydrocholesterol, cholestenone, lanosterol, 24,25-dihydrolanosterol.

5. Use according to claims 4, wherein one or a plurality of said target metabolites listed in claim 4 are suitable for an early diagnosis of sepsis.

6. Use according to anyone of claims 1 to 3, wherein said endogenous target metabolites are selected from the group consisting of:
22(R)-hydroxycholesterol, 24(S)-hydroxycholesterol, 25-hydroxycholesterol, 27-hydroxycholesterol, 3ß,5α,6ß-trihydroxycholestan, 7α-hydroxycholesterol, 7-ketocholesterol, 5ß,6ß-epoxycholesterol, 5α,6α-epoxycholesterol, 4ß-hydroxycholesterol, desmosterol, 7-dehydrocholesterol, cholestenone, lanosterol, 24,25-dihydrolanosterol.

7. Use according to claim 6, wherein one or a plurality of said target metabolites listed in claim 6 are suitable for an early diagnosis of a severe sepsis.

8. Use according to anyone of claims 1 to 3, wherein said endogenous target metabolites are selected from the group consisting of:
24(S)-hydroxycholesterol, 25-hydroxycholesterol, 27-hydroxycholesterol, 7-ketocholesterol, 5ß,6ß-epoxycholesterol, 5α,6α-epoxycholesterol, 4ß-hydroxycholesterol, desmosterol, lanosterol.

9. Use according to claim 8, wherein one or a plurality of said target metabolites listed in claim 8 are suitable for predicting a likelihood of an onset of a septic shock.

10. Use according to anyone of claims 1 to 9, wherein the biological sample is selected from the group consisting of stool; body fluids, in particular blood, liquor, cerebrospinal fluid, urine, ascitic fluid, seminal fluid, saliva, puncture fluid, cell content, tissue samples, in particular liver biopsy material; or a mixture thereof.

11. Use according to anyone of claims 2 to 10, wherein said quantitative metabolomics profile is achieved by a quantitative metabolomics profile analysis method comprising the generation of intensity data for the quantitation of endogenous metabolites by mass spectrometry (MS), in particular, by high-throughput mass spectrometry, preferably by MS-technologies such as Matrix Assisted Laser Desorption/lonisation (MALDl), Electro Spray Ionization (ESI), Atmospheric Pressure Chemical Ionization (APCl),¹H-, ¹³C- and/or ³¹P-Nuclear Magnetic Resonance spectroscopy (NMR), optionally coupled to MS, determination of metabolite concentrations by use of MS-technologies and/or methods coupled to separation, in particular Liquid Chromatography (LC-MS), Gas Chromatography (GC-MS), or Capillary Electrophoresis (CE-MS).
